(19) Europäisches Patentamt
European Patent Office
Office européen des brevets

(11) Publication number : **0 508 995 B1**

(12) **EUROPEAN PATENT SPECIFICATION**

(45) Date of publication of patent specification : **16.02.94 Bulletin 94/07**

(51) Int. Cl.$^5$ : **C07D 243/38,** C07D 401/12, C07D 405/12, C07D 471/04, A61K 31/55, // (C07D471/04, 243:00, 221:00)

(21) Application number : **91900776.5**

(22) Date of filing : **28.11.90**

(86) International application number : **PCT/EP90/02044**

(87) International publication number : **WO 91/10654 25.07.91 Gazette 91/17**

(54) **MUSCARINIC RECEPTOR ANTAGONISTS.**

(30) Priority : **06.01.90 GB 9000302**

(43) Date of publication of application : **21.10.92 Bulletin 92/43**

(45) Publication of the grant of the patent : **16.02.94 Bulletin 94/07**

(84) Designated Contracting States : **AT BE CH DE DK ES FR GB GR IT LI LU NL SE**

(56) References cited :
**EP-A- 0 346 744**
**GB-A- 1 581 500**

(73) Proprietor : **Pfizer Limited**
**Ramsgate Road**
**Sandwich Kent CT13 9NJ (GB)**
(84) **GB**
Proprietor : **PFIZER INC.**
**235 East 42nd Street**
**New York, N.Y. 10017 (US)**
(84) **BE CH DE DK ES FR GR IT LI LU NL SE AT**

(72) Inventor : **ALKER, David**
**Pfizer Central Research Ramsgate Road**
**Sandwich, Kent CT13 9NJ (GB)**
Inventor : **CROSS, Peter, Edward**
**Pfizer Central Research Ramsgate Road**
**Sandwich, Kent CT13 9NJ (GB)**

(74) Representative : **Wood, David John et al**
**PFIZER LIMITED, Ramsgate Road**
**Sandwich, Kent CT13 9NJ (GB)**

**EP 0 508 995 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to certain benzodiazepinone derivatives. The compounds of the invention are muscarinic receptor antagonists which are selective for smooth muscle muscarinic sites over cardiac muscarinic sites and which do not have any significant antihistaminic activity. Thus the compounds are useful in the treatment of diseases associated with altered motility and/or tone of smooth muscle which can, for example, be found in the gut, trachea and bladder. Such diseases include irritable bowel syndrome, diverticular disease, urinary incontinence, oesophageal achalasia and chronic obstructive airways disease.

According to the invention there are provided compounds of the formula:

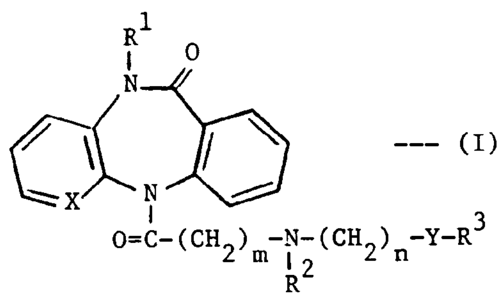

--- (I)

and their pharmaceutically acceptable salts,

wherein X is N or

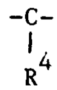

where

$R^4$ is H, halo or $C_1$-$C_4$ alkyl;

$R^1$ is H or $C_1$-$C_4$ alkyl;

$R^2$ is H or $C_1$-$C_4$ alkyl;

Y is a direct link, O or S;

m is an integer of from 1 to 4;

n is 2 or 3;

and $R^3$ is 1- or 2-naphthyl or a group of the formula:-

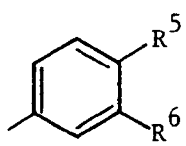

,

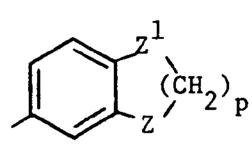

or Het

where $R^5$ and $R^6$ are each independently H, $C_1$-$C_4$ alkyl, $C_1$-$C_4$ alkoxy , $-(CH_2)_qOH$, halo, trifluoromethyl, cyano, $-(CH_2)_qNR^7R^8$, $-OCO(C_1$-$C_4$ alkyl$)$, $-SO_2NH_2$ or $-CONR^9R^{10}$;

where either $R^7$ and $R^8$ are each independently H or $C_1$-$C_4$ alkyl, or $R^7$ is H and $R^8$ is $-SO_2(C_1$-$C_4$alkyl$)$, $-CONR^9R^{10}$, $-CO(C_1$-$C_4$ alkyl$)$ or $SO_2NH_2$;

$R^9$ and $R^{10}$ are each independently H or $C_1$-$C_4$ alkyl;

q is 0, 1 or 2;

Z and $Z^1$ are each independently O or $CH_2$;

p is 1, 2 or 3;

and "Het" is pyridyl, pyrazinyl or thienyl.

Preferably, $R^1$ is H. X is preferably N or CH. m is preferably 1, 2 or 3. n is preferably 2. $R^2$ is preferably methyl. Y is preferably a direct link or O.

$R^3$ is preferably 2-naphthyl, pyridyl or a group of the formula:-

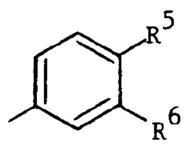

or

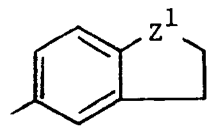

where $R^5$ and $R^6$ are each independently H, $C_1$-$C_4$ alkyl, $C_1$-$C_4$ alkoxy, halo (preferably chloro), trifluoromethyl, cyano or $C_1$-$C_4$ alkanesulphonamido, and $Z^1$ is O or $CH_2$.

The pharmaceutically acceptable salts of the compounds of formula (I) include acid addition salts such as the hydrochloride, hydrobromide, sulphate or bisulphate, phosphate or hydrogen phosphate, acetate, besylate, citrate, fumarate, gluconate, lactate, maleate, mesylate, succinate and tartrate salts. For a more comprehensive list of pharmaceutically acceptable salts see, for example, the Journal of Pharmaceutical Sciences, Vol. 66, No. 1, January 1977, pages 1-19. These salts can be prepared conventionally, e.g. by mixing a solution of the free base and the acid in a suitable solvent, e.g. ethanol, and recovering the acid addition salt either as a precipitate, or by evaporation of the solution.

The compounds of the formula (I) can be prepared by the following routes:-

Route A

This involves the reaction of a benzodiazepinone of the formula (II) with an alkylating agent of the formula (III), as follows:-

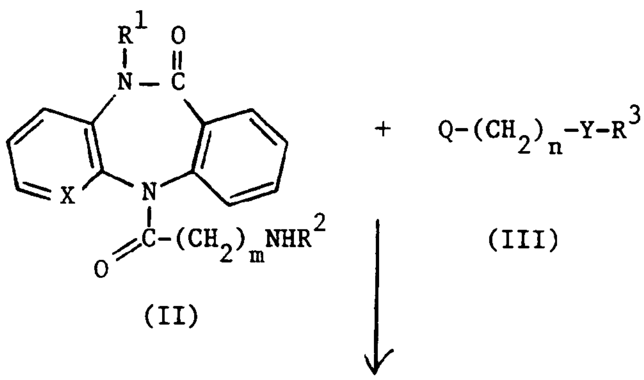

(II) (III)

Compounds (I)

In the above, X, Y, $R^1$, $R^2$, $R^3$, m and n are as defined for formula (I) and Q is a leaving group, e.g. Br, Cl, I, $C_1$-$C_4$ alkanesulfonyloxy (e.g. methanesulfonyloxy), benzenesulfonyloxy, toluenesulfonyloxy (e.g. p-toluenesulfonyloxy) or trifluoromethanesulfonyloxy. Preferably, Q is Cl, Br, I or methanesulfonyloxy. Most preferably, Q is Br.

The reaction is preferably carried out in the presence of an acid acceptor such as sodium hydrogen carbonate, sodium or potassium carbonate, triethylamine or pyridine, and in a suitable organic solvent, e.g. acetonitrile, at up to the reflux temperature. Reaction temperatures of 60-120°C are generally desirable and it is most convenient to carry out the reaction under reflux. Iodo is often a particularly suitable leaving group but since the starting materials (III) are sometimes most conveniently available as chlorides or bromides the reaction can also be carried out using the compound (III) as a chloride or bromide but in the presence of an iodide such as sodium or potassium iodide.

The starting materials of the formula (II) are either known compounds or can be prepared by conventional procedures, see e.g. J. Med. Chem., 1963, 6, 255, German Patentschrift no. 1,936,670, and British patent no. 1,581,500.

The starting materials of the formula (III) are again either known compounds or can be prepared conventionally: the preparation of any novel compounds of the formula (III) used in the Examples is in fact described in the following Preparations section.

Route B

This route can be represented schematically as follows:-

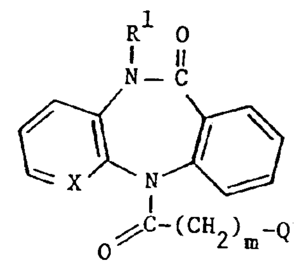

(IV)

+ $R^2NH-(CH_2)_n-Y-R^3$ → Compounds (I)

(VI)

or

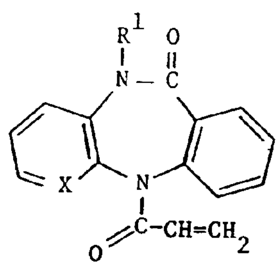

(V)

$R^1$, $R^2$, $R^3$, X, Y, m and n are as defined for formula (I) and Q is a leaving group such as is described in Route A. The reaction can be carried out similarly to Route A. Clearly use of the compound (V) will produce compounds (I) in which m is 2.

When m is 2, a mixture of the 11-(3-chloropropionyl) and 11-acryloyl compounds can be used: such a mixture is prepared in Preparation 1. Chromatographic techniques to separate the compounds can of course be used.

The compounds (IV) and (V) are either known (see e.g. GB 1,581,500 and DT-PS 1,936,670) or can be prepared by conventional techniques such as those described in the following Preparations 1 to 3.

The compounds (VI) are either known or can be prepared conventionally as is illustrated in the following Preparations.

The selectivity of the compounds as muscarinic receptor antagonists can be measured as follows.

Male guinea pigs are sacrificed and the ileum, trachea, bladder and right atrium are removed and suspended in physiological salt solution under a resting tension of 1 g at 32°C aerated with 95% $O_2$ and 5% $CO_2$. Contractions of the ileum, bladder and trachea are recorded using an isotonic (ileum) or isometric transducer (bladder and trachea). The frequency of contraction of the spontaneously beating right atrium is derived from isometrically recorded contractions.

Dose-response curves to either acetylcholine (ileum) or carbachol (trachea, bladder and right atrium) are determined using a 1-5 minute contact time for each dose of agonist until the maximum response is achieved. The organ bath is drained and refilled with physiological salt solution containing the lowest dose of the test compound. The test compound is allowed to equilibrate with the tissue for 20 minutes and the agonist dose-response curve is repeated until the maximum response is obtained. The organ bath is drained and refilled with physiological salt solution containing the second concentration of test compound and the above procedure is repeated. Typically four concentrations of the test compound are evaluated on each tissue.

The concentration of the test compound which causes a doubling of the agonist concentration required to produce the original response is determined ($pA_2$ value - Arunlakshana and Schild (1959), Brit. J. Pharmacol., 14, 48-58). Using the above analytical techniques, tissue selectivity for muscarinic receptor antagonists is determined.

Activity against agonist induced bronchoconstriction or gut or bladder contractility in comparison with

changes in heart rate is deterimined in the anaesthetised dog. Oral activity is assessed in the conscious dog determining compound effects on, for example, heart rate, pupil diameter and gut motility.

Compound affinity for other cholinergic sites is assessed in the mouse after either intravenous or intraperitoneal administration. Thus, the dose which causes a doubling of pupil size is determined as well as the dose which inhibits the salivation and tremor responses to intravenous oxotremorine by 50%.

For administration to man in the curative or prophylactic treatment of diseases associated with the altered motility and/or tone of smooth muscle, such as irritable bowel syndrome, diverticular disease, urinary incontinence, oesophageal achalasia and chronic obstructive airways disease, oral dosages of the compounds will generally be in the range of from 3.5 to 350 mg daily for an average adult patient (70 kg). Thus for a typical adult patient, individual tablets or capsules will typically contain from 1 to 250 mg of active compound, in a suitable pharmaceutically acceptable vehicle or carrier for administration singly or in multiple doses, once or several times a day. Dosages for intravenous administration will typically be within the range 0.35 to 35 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs of suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

In a further aspect the invention provides a pharmaceutical composition comprising a compound of the formula (I), or a pharmaceutically acceptable salt thereof, together with a pharmaceutically acceptable diluent or carrier.

The invention also includes a compound of the formula (I) or a pharmaceutically acceptable salt thereof, for use as a medicament, particularly for use in the treatment of irritable bowel syndrome.

The invention further includes the use of a compound of the formula (I), or of a pharmaceutically acceptable salt thereof, for the manufacture of a medicament for the treatment of diseases associated with the altered motility and/or tone of smooth muscle, such as irritable bowel syndrome, diverticular disease, urinary incontinence, oesophageal achalasia and chronic obstructive airways disease.

The invention yet further includes the use for compounds of formula (I) for preparing a medicament for the treatment of a human being to cure or prevent a disease associated with the altered motility and/or tone of smooth muscle, such as irritable bowel syndrome, which comprises treating said human being with an effective amount of a compound of the formula (I), or a pharmaceutically acceptable salt or composition thereof.

The Examples illustrate the preparation of the compounds of the formula (I), and the Preparations illustrate the preparation of certain of the starting materials used in the preceding Examples.

## EXAMPLE 1

5-{3-[N-(4-Methylphenethyl)-N-methylamino]propionyl}-10,11-dihydrodibenzo[b,e][1,4]diazepin-11-one

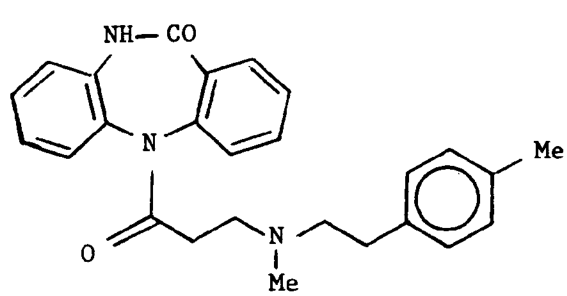

A mixture of 5-(3-methylaminopropionyl)-10,11-dihydrodibenzo[b,e][1,4]diazepin-11-one (0.20 g) (J. Med. Chem., 1963, 6, 255), 4-methylphenethyl bromide (0.14 g) and sodium hydrogen carbonate (60 mg) in acetonitrile (20 ml) was heated under reflux for 16 hours and evaporated. The residue was partitioned between water

and dichloromethane and the organic layer washed with brine, dried over $MgSO_4$ and evaporated. The residue was purified by chromatography on silica using dichloromethane plus 0-20% methanol as eluant. Appropriate fractions were combined and evaporated and the residue crystallised from ether to give the title compound as a colourless solid, 60 mg (21%).

Analysis %:-

| | |
|---|---|
| Found: | C,75.1; H,6.5; N,10.0; |
| $C_{26}H_{27}N_3O_2$ requires: | C,75.5; H,6.6; N,10.2. |

EXAMPLES 2-4

The following tabulated examples of the general formula:-

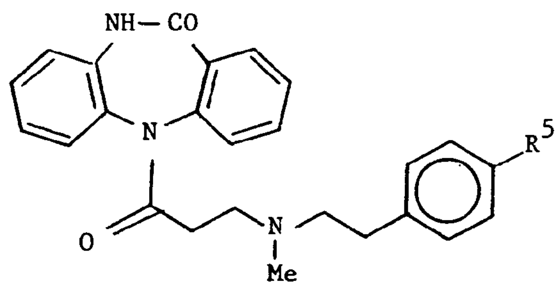

were prepared as described for Example 1 by reacting 5-(3-methylaminopropionyl)-10,11-dihydrodibenzo[b,e][1,4]-diazepin-11-one with a slight excess of the appropriate 2-arylethyl bromide in the presence of sodium hydrogen carbonate using acetonitrile as the solvent.

| Example | $R^5$ | Form characterised | Analysis % |
|---|---|---|---|
| 2 | -H | Colourless solid, m.p. 63-64°C | Found: C,74.7; H,6.4; N,10.3; $C_{25}H_{25}N_3O_2$ requires: C,75.2; H,6.3; N,10.5. |
| 3 | -Cl | Colourless solid, m.p. 68-70°C | Found: C,69.5; H,5.8; N,9.4; $C_{25}H_{24}ClN_3O_2$ requires: C,69.2; H,5.6; N,9.7. |
| 4 | $-OCH_3$ | Colourless oil. | Found: C,72.5; H,6.5; N,10.1; $C_{26}H_{27}N_3O_3$ requires: C,72.7; H,6.3; N,9.8. |

EXAMPLE 5

5,11-Dihydro-11-{2-[N-(4-methoxyphenethyl)-N-methylamino]acetyl} -6H-pyrido[2,3-b][1,4]benzodiazepin-6-one

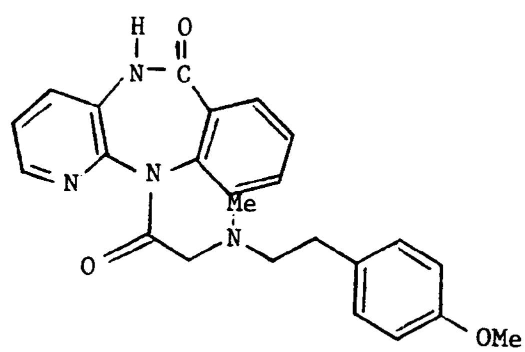

A mixture of 11-chloroacetyl-5,11-dihydro-6H-pyrido- [2,3-b][1,4]benzodiazepine-6-one (288 mg) (German patent 1,936,670), N-(4-methoxyphenethyl)methylamine (182 mg) and sodium hydrogen carbonate (92 mg) in acetonitrile (25 ml) was heated under reflux for 16 hours and evaporated. The residue was partitioned between 2M aqueous sodium hydrogen carbonate solution and dichloromethane and the organic layer washed with brine, dried over $MgSO_4$ and evaporated. The residue was purified by chromatography on silica using dichloromethane plus 0-10% methanol as eluant. Appropriate fractions were combined and evaporated and the residue crystallised from ethyl acetate to give the title compound as a colourless solid, 216 mg (52%).

Analysis %:-

| | |
|---|---|
| Found: | C,69.2; H,5.8; N,13.5; |
| $C_{24}H_{24}N_4O_3$ requires: | C,69.2; H,5.8; N,13.4. |

EXAMPLES 6-10

The following tabulated Examples of the general formula:-

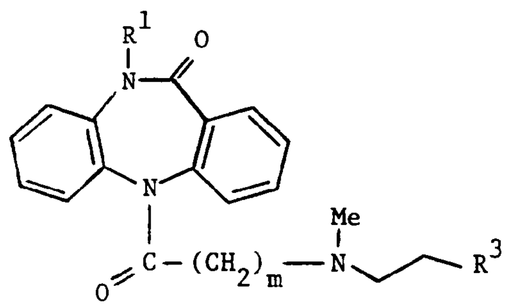

were prepared as described for Example 5 by reacting the appropriate 5-chloroacyl-10,11-dihydrodibenzo[b,e][1,4]diazepin-6-one with one equivalent of the appropriate arylalkylmethylamine in the presence of two equivalents of sodium hydrogen carbonate using acetonitrile as the solvent. The product of Example 10 was characterised as containing 0.10 equivalents of dichloromethane (derived from the chromatography) while the product of Example 8 was characterised as a hydrate. The preparation of the starting material for Examples 6-8 is described in Preparation 2 while the preparation of the starting material for Example 9 is described in Preparation 3. The starting material for Example 10 was prepared according to German patent no. 1,936,670.

| Example No. | $R^1$ | m | $R^3$ | Form characterised | Analysis % |
|---|---|---|---|---|---|
| 6 | H | 3 | 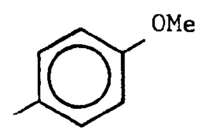 | Colourless gum | Characterised by $^1$H-N.M.R. ($CDCl_3$) $\delta$ = 7.1-8.3 (m, 8H); 7.13 (d, J = 8Hz, 2H); 6.83 (d, J = 8Hz, 2H); 3.81 (s, 3H); 2.1-2.8 (m, 8H); 2.24 (s, 3H); 1.65-1.95 (m, 2H). |
| 7 | H | 3 | 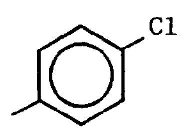 | Colourless foam | Found: C,69.7; H,5.9; H,9.4; Calculated for $C_{26}H_{26}ClN_3O_2$: C,69.3; H,5.8; N,9.4. |
| 8 | H | 3 | 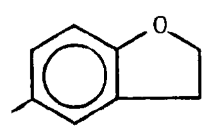 | Colourless oil; hydrate | Found: C,71.2; H,6.5; N,9.1; Calculated for $C_{28}H_{29}N_3O_3.H_2O$: C,71.0; H,6.6; N,8.8. |

| Example No. | $R^1$ | m | $R^3$ | Form characterised | Analysis % |
|---|---|---|---|---|---|
| 9 | Me | 3 | 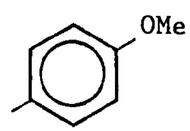 | Colourless oil | Found: C,73.5; H,7.2; N,9.0; Calculated for $C_{28}H_{31}N_3O_3$: C,73.5; H,6.8; N,9.2. |
| 10 | Me | 2 | 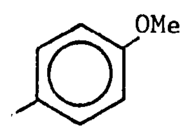 | Colourless oil containing 0.10 equivalents of dichloromethane | Found: C,71.9; H,6.6; N,9.3; Calculated for $C_{27}H_{29}N_3O_3 \cdot 0.1\ CH_2Cl_2$: C,72.0; H,6.5; N,9.3. |

EXAMPLE 11

5,11-Dihydro-11-[3-[N-(4-methoxyphenethyl)-N-methylamino]-propionyl-6H-pyrido[2,3-b][1,4]benzodiazepin-6-one

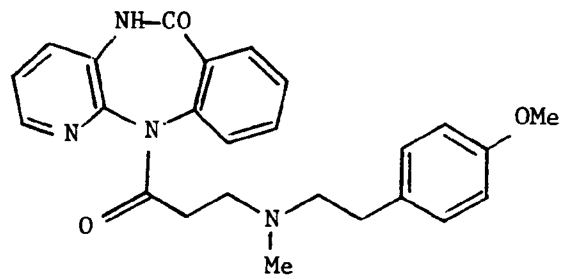

A mixture of 11-acryloyl-5,11-dihydro-6H-pyrido[2,3-b]-[1,4]benzodiazepin-6-one (185 mg) (see Preparation 1) and N-(4-methoxyphenethyl)methylamine (127 mg) in dioxane (15 ml) was heated under reflux for 4 hours and evaporated. The residue was partitioned between water and dichloromethane and the organic layer dried over $MgSO_4$ and evaporated. The residue was purified by chromatography on silica using dichloromethane plus 0-2% methanol as eluant. Appropriate fractions were combined and evaporated to give the title compound as a pale yellow solid, 182 mg (60%), m.p. 163-164°C, which was characterised as a hemihydrate.

Analysis %:-

| | |
|---|---|
| Found: | C,68.0; H,6.2; N,12.6; |
| $C_{25}H_{26}N_4O_3$.0.5 $H_2O$ requires: | C,68.3; H,6.2; N,12.7. |

EXAMPLES 12-21

The following tabulated Examples of the general formula:-

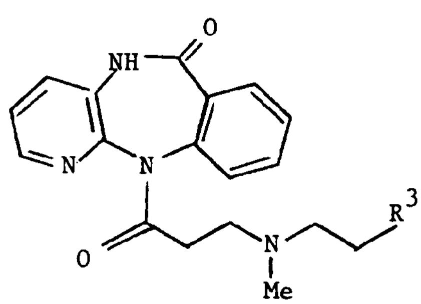

were prepared as described for Example 11 by reacting 11-acryloyl-5,11-dihydro-6H-pyrido[2,3-b][1,4]benzodiazepin-6-one (see Preparation 1) with a slight excess of the appropriate arylalkylmethylamine in the presence of excess sodium hydrogen carbonate using acetonitrile as solvent. The products of Examples 12, 13, 16, 18 and 19 were characterised as hemihydrates.

| Example No | $R^3$ | Form characterised | Analysis % |
|---|---|---|---|
| 12 | 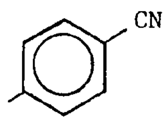 | Colourless solid, m.p. 82-83°C, hemihydrate | Found: C,69.8; H,5.5; N,14.9; $C_{25}H_{23}N_5O_2.0.5\ H_2O$ requires: C,69.1; H,5.6; N,16.1. |
| 13 | 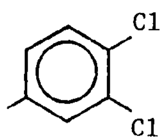 | Colourless solid, m.p. 170°C, hemihydrate | Found: C,59.8; H,4.6; N,11.4; $C_{24}H_{22}Cl_2N_4O_2.0.5\ H_2O$ requires: C,60.2; H,4.8; N,11.7. |
| 14 | 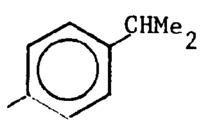 | Colourless solid, m.p. 66°C | Found: C,73.4; H,6.8; N,12.7; $C_{27}H_{30}N_4O_2$ requires: C,73.3; H,6.8; N,12.7. |
| 15 | 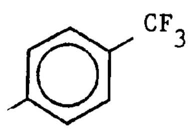 | Colourless solid, m.p. 66-68°C | Found: C,64.2; H,4.9; N,12.0; $C_{25}H_{23}F_3N_4O_2$ requires: C,64.1; H,4.9; N,12.0. |

| Example No | $R^3$ | Form characterised | Analysis % |
|---|---|---|---|
| 16 | 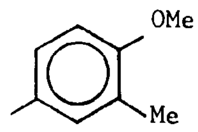 | Colourless solid, m.p. 70-72°C, hemihydrate | Found: C,68.7; H,6.3; N,12.3; $C_{26}H_{28}N_4O_3$.0.5 $H_2O$ requires: C,68.7; H,6.4; N,12.3. |
| 17 | 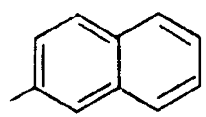 | Colourless solid, m.p. 168-170°C | Found: C,73.4; H,5.8; N,12.2; $C_{28}H_{26}N_4O_2$ requires: C,73.2; H,5.9; N,12.2. |
| 18 | 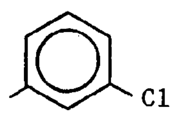 | Colourless solid, m.p. 69-71°C, hemihydrate | Found: C,65.4; H,5.3; N,12.8; $C_{24}H_{23}ClN_4O_2$.0.5 $H_2O$ requires: C,64.9; H,5.4; N,12.6. |
| 19 | 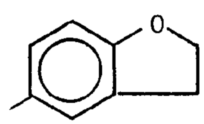 | Colourless solid, m.p. 155-157°C, hemihydrate | Found: C,69.5; H,6.3; N,12.3; $C_{26}H_{26}N_4O_3$.0.5 $H_2O$ requires: C,69.2; H,6.0; N,12.4. |

| Example No | $R^3$ | Form characterised | Analysis % |
| --- | --- | --- | --- |
| 20 | 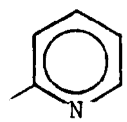 | Colourless solid, m.p. 63-65°C | Found: C,68.9; H,5.9; N,17.4; $C_{23}H_{23}N_5O_2$ requires: C,68.8; H,5.8; N,17.4. |
| 21 | 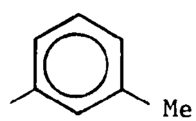 | Colourless solid, m.p. 141-142°C | Found: C,72.5; H,6.4; N,13.4; $C_{25}H_{26}N_4O_2$ requires: C,72.4; H,6.3; N,13.5. |

EXAMPLES 22-30

The following tabulated Examples of the general formula:-

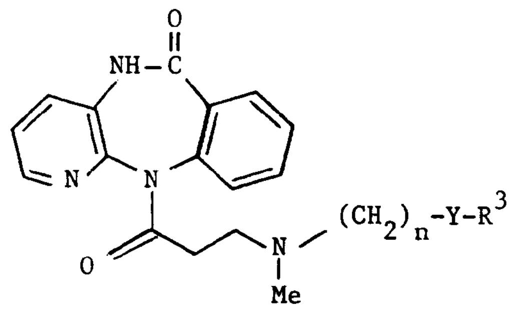

were prepared as described for Example 11 by reacting a mixture of 11-acryloyl-5,11-dihydro-6H-pyrido[2,3-b][1,4]benzodiazepin-6-one and 11-(3-chloropropionyl)-5,11-dihydro-6H-pyrido[2,3-b][1,4]-benzodiazepin-6-one (see Preparation 1) with a slight excess of the appropriate arylalkylmethylamine in the presence of excess sodium hydrogen carbonate using acetonitrile as solvent. The products of Examples 23-28 were characterised as hemihydrates. The N-(4-methanesulphonamidophenethyl)methylamine used in Example 30 was prepared as described in EP-A-245,997.

| Example No. | Y | $R^3$ | n | Form characterised | Analysis % |
|---|---|---|---|---|---|
| 22 | a direct link | 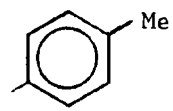 | 2 | Colourless solid, m.p. 163-164°C | Found: C,72.1; H,6.3; N,13.7; $C_{25}H_{26}N_4O_2$ requires: C,72.4; H,6.3; N,13.5. |
| 23 | a direct link | 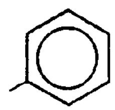 | 2 | Colourless solid, m.p. 144-145°C hemihydrate | Found: C,71.1; H,6.3; N,13.1; $C_{25}H_{26}N_4O_2$.0.5 $H_2O$ requires: C,70.9; H,6.4; N,13.2. |
| 24 | a direct link | 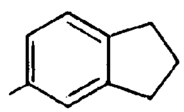 | 2 | Colourless solid, m.p. 171-172°C, hemihydrate | Found: C,72.3; H,6.5; N,12.3; $C_{27}H_{28}N_4O_2$.0.5 $H_2O$ requires: C,72.1; H,6.5; N,12.5. |
| 25 | O | 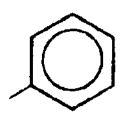 | 2 | Colourless solid, m.p. 67°C, hemihydrate | Found: C,68.0; H,5.9; N,13.4; $C_{24}H_{24}N_4O_3$.0.5 $H_2O$ requires: C,67.8; H,5.9; N,13.2. |

| Example No. | Y | $R^3$ | n | Form characterised | Analysis % |
|---|---|---|---|---|---|
| 26 | a direct link | 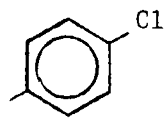 | 2 | Colourless solid, m.p. 150-151°C, hemihydrate | Found: C,65.3; H,5.2; N,12.6; $C_{24}H_{23}ClN_4O_2 \cdot 0.5\ H_2O$ requires: C,64.9; H,5.4; N,12.6. |
| 27 | a direct link | 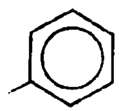 | 2 | Colourless solid, m.p. 144°C, hemihydrate | Found: C,70.6; H,6.0; N,13.5; $C_{24}H_{24}N_4O_2 \cdot 0.5\ H_2O$ requires: C,70.4; H,6.1; N,13.7. |
| 28 | a direct link | 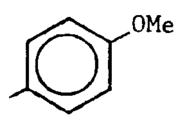 | 3 | Colourless solid, m.p. 48-50°C, hemihydrate | Found: C,68.6; H,6.1; N,12.3; $C_{26}H_{28}N_4O_3 \cdot 0.5\ H_2O$ requires: C,68.8; H,6.4; N,12.3. |
| 29 | O | 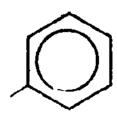 | 3 | Colourless solid, m.p. 65-67°C | Found: C,69.2; H,6.1; N,13.0; $C_{25}H_{26}N_4O_3$ requires: C,69.7; H,6.1; N,13.0. |

| Example No. | Y | $R^3$ | n | Form characterised | Analysis % |
|---|---|---|---|---|---|
| 30 | a direct link | 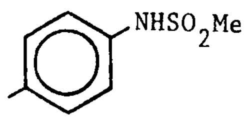 | 2 | Colourless solid, m.p. 184-185°C | Found: C,60.7; H,5.4; N,14.1; $C_{25}H_{27}N_5O_4S$ requires: C,60.8; H,5.5; N,14.2. |

The following Preparations illustrate the preparation of certain of the starting materials used in the previous Examples.

Preparation 1

11-Acryloyl-5,11-dihydro-6H-pyrido[2,3-b][1,4]benzodiazepin-6-one and a mixture thereof with the corresponding 11-(3-chloropropionyl) compound

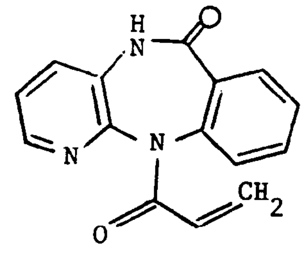

Solutions of 3-chloropropionyl chloride (6.3 g) in dioxane (60 ml) and triethylamine (8.4 g) in dioxane (60 ml) were added simultaneously to a refluxing suspension of 5,11-dihydro-6H-pyrido[2,3-b][1,4]benzodiazepin-6-one (9.45 g - see DT-PS 1179943) in dioxane (300 ml) and the mixture was heated under reflux for 6 hours and evaporated to give a mixture of the two title compounds in which the acryloyl compound predominated. Crude mixtures of the 11-acryloyl and 11-(3-chloropropionyl) compounds prepared using this procedure were used in Examples 22-30. The residue was purified by chromatography on silica using dichloromethane plus 0-2% methanol as eluant. Appropriate fractions were combined and evaporated to give the title 11-acryloyl compound as a colourless solid, 3.2 g, (27%) which was used directly in Examples 11-21.

Preparation 2

5-(4-Chlorobutyryl)-10,11-dihydrodibenzo[b,e][1,4]diazepin-11-one

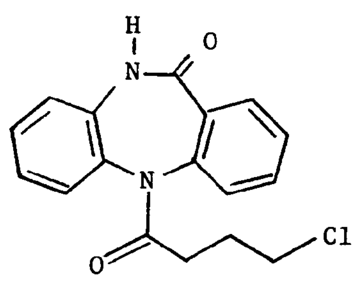

A mixture of 4-chlorobutyryl chloride (3.5 g) and 10,11-dihydrodibenzo[b,e][1,4]diazepin-11-one (4.2 g) (J. Med. Chem., 1963, 6, 767) in acetone (90 ml) was heated under reflux for 8 hours and evaporated. The residue was purified by chromatography on silica using hexane plus 0-100% dichloromethane as eluant. Appropriate fractions were combined and evaporated and the residue triturated with hexane/dichloromethane to give the title compound as a colourless solid, 1.62 g (26%), m.p. 151-152°C.

Analysis %:-

| | |
|---|---|
| Found: | C,64.6; H,4.7; N,8.8; |
| $C_{17}H_{15}ClN_2O_2$ requires: | C,64.9; H,4.8; N,8.9. |

Preparation 3

5-(4-Chlorobutyryl)-10,11-dihydro-10-methyldibenzo[b,e][1,4]- diazepin-11-one

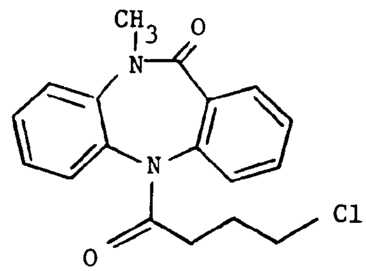

A mixture of 4-chlorobutyryl chloride (0.88 g) and 10,11-dihydro-10-methyldibenzo[b,e][1,4]diazepin-10-one (1.12 g) (J. Med. Chem., 1963, 6, 767) in acetone (25 ml) was heated under reflux for 4 hours and evaporated. The residue was dissolved in ethyl acetate and the solution washed with 10% aqueous sodium hydrogen carbonate solution, dried over $Na_2SO_4$ and evaporated. The residue was purified by chromatography on silica using hexane plus 0-100% dichloromethane as eluant. Appropriate fractions were combined and evaporated to give the title compound as a colourless oil, 1.10 g (67%).

Analysis %:-

| | |
|---|---|
| Found: | C,65.9; H,5.4; N,8.4; |
| $C_{18}H_{17}ClN_2O_2$ requires: | C,65.7; H,5.2; N,8.5. |

Preparation 4

N-(3-Methylphenethyl)methylamine

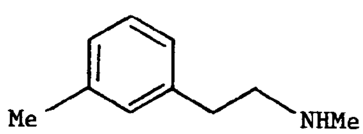

A mixture of 3-methylphenethyl bromide (2.22 g) and 33% ethanolic methylamine solution (30 ml) was heated in a bomb at 80°C for 16 hours and evaporated. The residue was partitioned between water and dichloromethane and the organic layer dried over $MgSO_4$ and evaporated. The residue was purified by chromatography on silica using dichloromethane plus 0-10% methanol as eluant. Appropriate fractions were combined and evaporated to give the title compound as a colourless oil, 0.44 g (27%), which was used directly in the preparation of Example 21 without characterisation.

Preparations 5-10

The following tabulated Preparations of the general formula:-

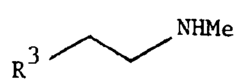

were prepared as described for Preparation 4 by reacting the appropriate arylethyl bromide with 33% ethanolic methylamine solution. In each case the product was characterised by its $^1$H-N.M.R. spectrum. The preparation of the starting materials for Preparations 7, 10, 6 and 8 are described in Preparations 11, 12, 13 and 14. The product from Preparation 10 was obtained as a colourless solid.

| Preparation No. | $R^3$ | Form characterised | $^1$H-N.M.R. |
|---|---|---|---|
| 5 | 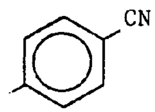 | Yellow oil | $^1$H-N.M.R. ($CDCl_3$) $\delta$ = 7.59 (d, J = 8Hz, 2H); 7.30 (d, J = 8Hz, 2H); 2.86 (s, 4H); 2.63 (s, 3H); 1.22 (s, 1H). |
| 6 | 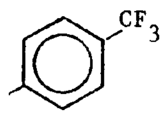 | Yellow oil | $^1$H-N.M.R. ($CDCl_3$) $\delta$ = 7.58 (d, J = 8Hz, 2H); 7.36 (d, J = 8Hz, 2H); 2.85 (s, 4H); 2.44 (s, 3H); 1.37 (s, 1H). |
| 7 | 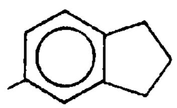 | Dark oil | $^1$H-N.M.R. ($CDCl_3$) $\delta$ = 6.8-7.4 (m, 3H); 2.60-3.05 (m, 4H); 2.43 (s, 3H); 2.05 (t, J = 7Hz, 2H); 1.90 (s, 1H). |
| 8 | 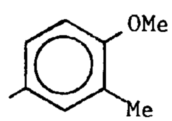 | Yellow oil | $^1$H-N.M.R. ($CDCl_3$) $\delta$ = 6.97-7.08 (m, 2H); 6.79 (d, J = 8Hz, 1H); 4.92 (broad s, 1H); 3.81 (s, 3H); 2.88-3.00 (m, 4H); 2.57 (s, 3H); 2.21 (s, 3H). |
| 9 | 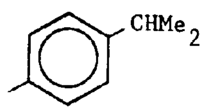 | Yellow oil | $^1$H-N.M.R. ($CDCl_3$) $\delta$ = 7.06-7.24 (m, 4H); 2.80-3.00 (m, 5H); 2.46 (s, 3H); 2.06 (broad s, 1H); 1.24 (d, J = 7Hz, 6H). |
| 10 | 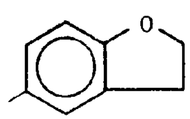 | Colourless solid, m.p. 153-155°C. | $^1$H-N.M.R. ($CDCl_3$) $\delta$ = 7.11 (s, 1H); 6.99 (d, J = 8Hz, 1H); 6.77 (d, J = 8Hz, 1H); 4.58 (t, J = 7Hz, 2H); 3.13 (s, 4H); 3.11 (t, J = 7Hz, 2H); 1.13 (broad s, 1H). |

Preparation 11

5-(2-Bromoethyl)indane

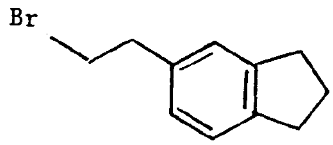

Phosphorus tribromide (3.5 ml) was added, dropwise, to a solution of 5-(2-hydroxyethyl)indane (14.0 g) (FR-A-2139628) in carbon tetrachloride (100 ml). The mixture was stirred at room temperature for 0.5 hour and then heated under reflux for 2 hours. Ice (100 g) was added and the mixture partitioned between dichloromethane and 10% aqueous sodium carbonate solution. The layers were separated and the aqueous layer extracted wilth dichloromethane (2 x 100 ml). The combined dichloromethane extracts were dried ($MgSO_4$) and concentrated in vacuo to give an oil which was purified by column chromatography on silica eluting with dichloromethane. The product-containing fractions were combined and concentrated in vacuo to give the title compound as a colourless oil, yield 10.5 g.
$^1$H N.M.R. ($CDCl_3$) $\delta$ = 7.00-7.30 (m, 3H); 3.60 (m, 2H); 3.20 (m, 2H); 2.85-3.00 (m, 4H); 2.05-2.20 (m, 2H).

Preparation 12-14

The following tabulated Preparations of the general formula:-

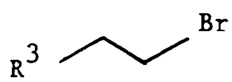

were prepared as described for Preparation 11 by reacting the appropriate arylethyl alcohol with phosphorus tribromide in carbon tetrachloride solution. In each case the product was obtained as a yellow oil which was characterised by its $^1$H-N.M.R. spectrum. The preparation of the starting materials for Preparations 12 and 13 are described in Preparations 15 and 16, respectively.

| Preparation No. | $R^3$ | $^1$H-N.M.R. ($CDCl_3$), $\delta$ |
|---|---|---|
| 12 | 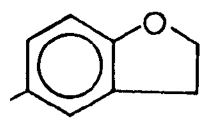 | 7.10 (s, 1H); 6.95-7.00 (d, 1H); 6.70-6.80 (d, 1H); 4.60 (t, J = 7Hz, 2H); 3.55 (t, J = 7Hz, 2H); 3.20 (t, J = 7Hz, 2H); 3.12 (t, J = 7Hz, 2H). |
| 13 | 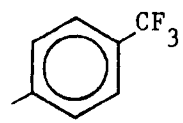 | 7.62 (d, J = 8Hz, 2H); 7.39 (d, J = 8Hz, 2H); 3.63 (t, J = 7Hz, 2H); 3.24 (t, J = 7Hz, 2H). |
| 14 | 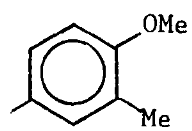 | 7.01-7.07 (m, 2H); 6.79 (d, J = 8Hz, 2H); 3.84 (s, 3H); 3.58 (t, J = 7Hz, 2H); 3.10 (t, J = 7Hz, 2H); 2.22 (s, 3H). |

Preparation 15

5-(2-Hydroxyethyl)-2,3-dihydrobenzofuran

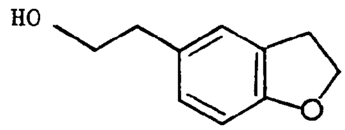

A solution of (2,3-dihydrobenzofuran-5-yl)acetic acid (4.9 g - see EP-A-132130) in anhydrous tetrahydrofuran (50 ml) was added dropwise over 10 minutes to a stirred suspension of lithium aluminium hydride (1.57 g) in anhydrous tetrahydrofuran (50 ml) at 0°C. The mixture was allowed to warm to room temperature and stirred for 1 hour. Water (1.5 ml) was cautiously added dropwise followed by 10% aqueous sodium hydroxide (1.5 ml) and, finally, water (4.5 ml). The mixture was filtered and the inorganic salts washed with ethyl acetate (2 x 50 ml). The filtrate and washings were combined and concentrated in vacuo to give the title compound as an oil, yield 3.3 g.
$^1$H N.M.R. ($CDCl_3$) $\delta$ = 7.10 (s, 1H); 7.00 (d, J = 8Hz, 1H); 6.75 (m, 1H); 4.55-4.65 (m, 2H); 3.75-3.90 (m, 2H); 3.15-3.30 (m, 2H); 2.80-2.90 (m, 2H); 1.75-1.85 (broad s, 1H).

Preparation 16

4-Trifluoromethylphenethyl alcohol

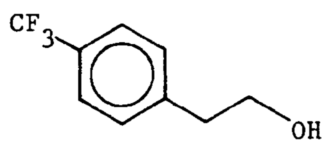

This was obtained by method described in Preparation 15 using 4-trifluoromethylphenylacetic acid instead of (2,3-dihydrobenzofuran-5-yl)acetic acid as the starting material. The title compound was obtained as a colourless oil, 3.75 g (80%), which was characterised by its $^1$H-N.M.R. spectrum.
$^1$H-N.M.R. ($CDCl_3$) $\delta$ = 7.59 (d, J = 8Hz, 2H); 7.38 (d, J = 8Hz, 2H); 3.94 (t, J = 7Hz, 2H); 2.97 (t, J = 7Hz, 2H); 1.62 (s, 1H).

## Claims

**Claims for the following Contracting States : AT, BE, CH, DE, DK, FR, GB, IT, LI, LU, NL, SE**

1. A compound of the formula:

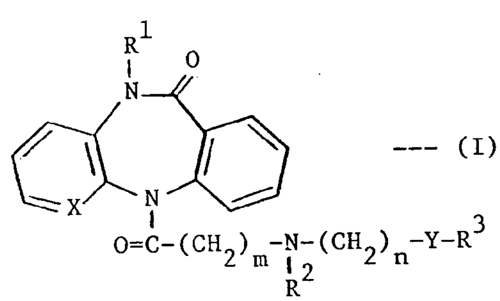

--- (I)

or a pharmaceutically acceptable salt thereof,
wherein X is N or

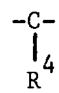

where
$R^4$ is H, halo or $C_1$-$C_4$ alkyl;
$R^1$ is H or $C_1$-$C_4$ alkyl;
$R^2$ is H or $C_1$-$C_4$ alkyl;
Y is a direct link, O or S;
m is an integer of from 1 to 4;
n is 2 or 3;
and $R^3$ is 1- or 2-naphthyl or a group of the formula:-

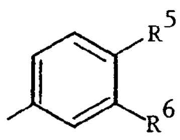
,
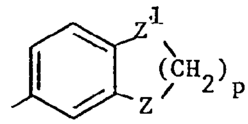

or Het

where $R^5$ and $R^6$ are each independently H, $C_1$-$C_4$ alkyl, $C_1$-$C_4$ alkoxy , $(CH_2)_qOH$, halo, trifluoromethyl, cyano, $-(CH_2)_qNR^7R^8$, $-OCO(C_1$-$C_4$ alkyl), $-SO_2NH_7$ or $-CONR^9R^{10}$;
where either $R^7$ and $R^8$ are each independently H or $C_1$-$C_4$ alkyl, or $R^7$ is H and $R^8$ is $-SO_2(C_1$-$C_4$ alkyl), $-CONR^9R^{10}$, $-CO(C_1$-$C_4$ alkyl) or $-SO_2NH_2$;
$R^9$ and $R^{10}$ are each independently H or $C_1$-$C_4$ alkyl;
q is 0, 1 or 2;
Z and $Z^1$ are each independently O or $CH_2$;
p is 1, 2 or 3;
and "Het" is pyridyl, pyrazinyl or thienyl.

2. A compound as claimed in claim 1 wherein $R^1$ is H, X is N or CH, m is 1, 2 or 3, n is 2, $R^2$ is methyl and Y is a direct link or O.

3. A compound as claimed in claim 1 or 2 wherein $R^3$ is 2-naphthyl, pyridyl or a group of the formula:-

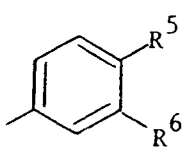
or
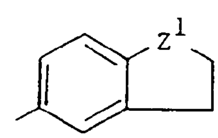

where $R^5$ and $R^6$ are each independently H, $C_1$-$C_4$ alkyl, $C_1$-$C_4$ alkoxy, halo, trifluoromethyl, cyano or $C_1$-$C_4$ alkanesulphonamido, and $Z^1$ is O or $CH_2$.

4. A pharmaceutical composition comprising a compound of the formula (I) as claimed in any one of the preceding claims, or a pharmaceutically acceptable salt thereof, and a pharmaceutically acceptable diluent or carrier.

5. A compound of the formula (I) as claimed in any one of claims 1 to 3, or a pharmaceutically acceptable salt thereof, for use as a medicament.

6. The use of a compound of the formula (I) as claimed in any one of claims 1 to 3, or of a pharmaceutically acceptable salt thereof, for the manufacture of a medicament for use as a muscarinic receptor antagonist.

7. A process for preparing a compound of the formula:-

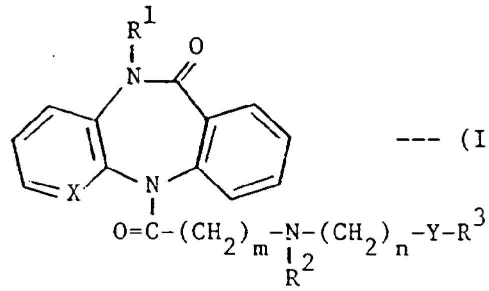

--- (I)

or a pharmaceutically acceptable salt thereof,

wherein X is N or

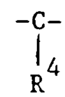

where
$R^4$ is H, halo or $C_1$-$C_4$ alkyl;
$R^1$ is H or $C_1$-$C_4$ alkyl;
$R^2$ is H or $C_1$-$C_4$ alkyl;
Y is a direct link, O or S;
m is an integer of from 1 to 4;
n is 2 or 3;

and $R^3$ is 1- or 2-naphthyl or a group of the formula:-

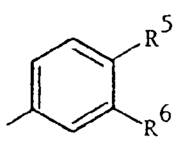

,

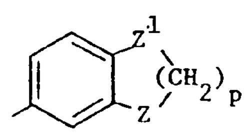

or Het

where $R^5$ and $R^6$ are each independently H, $C_1$-$C_4$ alkyl, $C_1$-$C_4$ alkoxy , $-(CH_2)_qOH$, halo, trifluoromethyl, cyano, $-(CH_2)_qNR^7R^8$, $-OCO(C_1$-$C_4$ alkyl), $-SO_2NH_2$ or $-CONR^9R^{10}$;

where either $R^7$ and $R^8$ are each independently H or $C_1$-$C_4$ alkyl, or $R^7$ is H and $R^8$ is $-SO_2(C_1$-$C_4$ alkyl), $-CONR^9R^{10}$, $-CO(C_1$-$C_4$ alkyl) or $-SO_2NH_2$;
$R^9$ and $R^{10}$ are each independently H or $C_1$-$C_4$ alkyl;
q is 0, 1 or 2;
Z and $Z^1$ are each independently O or $CH_2$,
p is 1, 2 or 3;

and "Het" is pyridyl, pyrazinyl or thienyl;

characterised by either:-

(a) reacting a compound of the formula:-

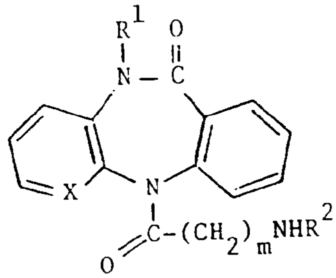

--- (II)

where $R^1$, $R^2$, X and m are as defined above, with a compound of the formula:-

$$Q-(CH_2)_n-Y-R^3 \quad (III)$$

where $R^3$, Y and n are as defined above and Q is a leaving group, or

(b) reacting a compound of the formula (IV) or (V)

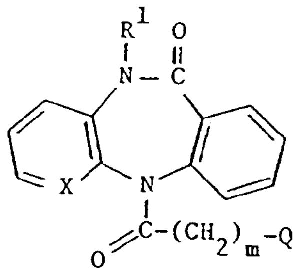

(IV)

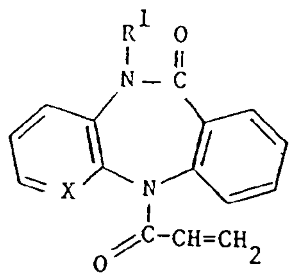

(V)

where $R^1$, X and m are as defined above and Q is a leaving group, with a compound of the formula:-

$$R^2NH-(CH_2)_n-Y-R^3 \quad (VI)$$

where $R^2$ $R^3$ Y and n are as defined above,

said processes (a) and (b) being followed by, optionally, conversion of the product (I) into a pharmaceutically acceptable salt.

**8.** A process according to claim 7, characterised in that Q is Cl, Br, I or methanesulfonyloxy, and in that in (a) and (b) the reactions are carried out in the presence of an acid acceptor.

**9.** The use of compounds of formula (I) for the preparation of a medicament for treating bowel syndrome in a patient in need of such treatment, characterised by administering to said patient an effective amount of a compound of the formula (I) or pharmaceutically acceptable salt thereof as claimed in any one of claims 1 to 3.

**Claims for the following Contracting States : ES, GR**

**1.** A process for preparing a compound of the formula:-

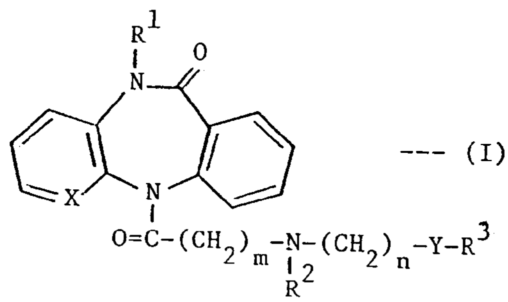

--- (I)

or a pharmaceutically acceptable salt thereof,

wherein X is N or

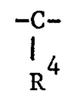

where

$R^4$ is H, halo or $C_1$-$C_4$ alkyl;

$R^1$ is H or $C_1$-$C_4$ alkyl;

$R^2$ is H or $C_1$-$C_4$ alkyl;

Y is a direct link, O or S;

m is an integer of from 1 to 4;

n is 2 or 3;

and $R^3$ is 1- or 2-naphthyl or a group of the formula:-

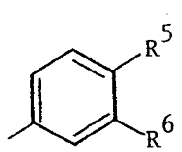

,

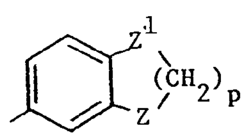

or Het

where $R^5$ and $R^6$ are each independently H, $C_1$-$C_4$ alkyl, $C_1$-$C_4$ alkoxy , $(CH_2)_qOH$, halo, trifluoromethyl, cyano, $(CH_2)_qNR^7R^8$, -OCO($C_1$-$C_4$ alkyl), $-SO_2NH_2$ or $-CONR^9R^{10}$;

where either $R^7$ and $R^8$ are each independently H or $C_1$-$C_4$ alkyl, or $R^7$ is H and $R^8$ is $-SO_2$($C_1$-$C_4$ alkyl), $-CONR^9R^{10}$, -CO($C_1$-$C_4$ alkyl) or $-SO_2NH_2$;

$R^9$ and $R^{10}$ are each independently H or $C_1$-$C_4$ alkyl;

q is 0, 1 or 2;

Z and $Z^1$ are each independently O or $CH_2$;

p is 1, 2 or 3;

and "Het" is pyridyl, pyrazinyl or thienyl;

characterised by either:-

(a) reacting a compound of the formula:-

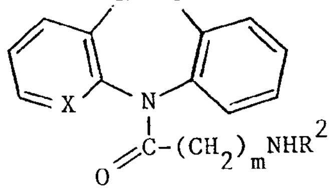

--- (II)

where $R^1$, $R^2$, X and m are as defined above,
with a compound of the formula:-

$$Q\text{-}(CH_2)_n\text{-}Y\text{-}R^3 \qquad (III)$$

where $R^3$, Y and n are as defined above and Q is a leaving group,
or
(b) reacting a compound of the formula (IV) or (V)

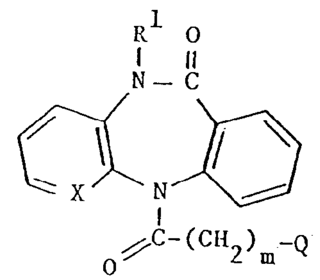

(IV)

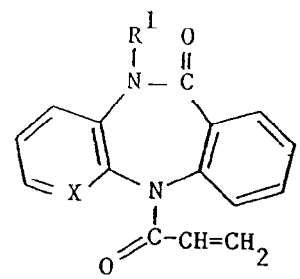

(V)

where $R^1$, X and m are as defined above and Q is a leaving group, with a compound of the formula:-

$$R^2NH\text{-}(CH_2)_n\text{-}Y\text{-}R^3 \qquad (VI)$$

where $R^2$, $R^3$, Y and n are as defined above,
said processes (a) and (b) being followed by, optionally, conversion of the product (I) into a pharmaceutically acceptable salt.

2. A process according to claim 1, characterised in that Q is Cl, Br, I or methanesulfonyloxy, and in that in (a) and (b) the reactions are carried out in the presence of an acid acceptor.

3. A process according to claim 1 or 2, characterised in that it is used to prepare a compound of the formula (I) in which $R^1$ is H, X is N or CH, m is 1, 2 or 3, n is 2, $R^2$ is methyl, Y is a direct link or 0, and $R^3$ is 2-naphthyl, pyridyl or a group of the formula:-

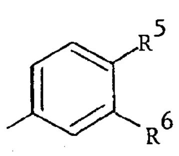

or

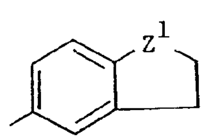

where $R^5$ and $R^6$ are each independently H, $C_1$-$C_4$ alkyl, $C_1$-$C_4$ alkoxy, halo, trifluoromethyl, cyano or $C_1$-$C_4$ alkanesulphonamido, and $Z^1$ is O or $CH_2$.

## Patentansprüche

### Patentansprüche für folgende Vertragsstaaten : AT, BE, CH, DE, DK, FR, GB, IT, LI, LU, NL, SE

**1.** Verbindung der Formel:

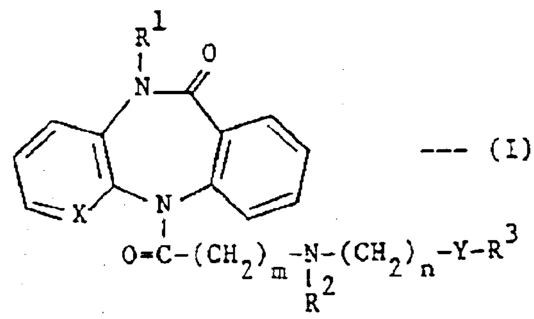

--- (I)

oder ein pharmazeutisch verträgliches Salz davon, worin X N oder

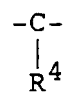

bedeutet, wobei

$R^4$ H, Halogen oder $C_1$-$C_4$-Alkyl darstellt;
$R^1$ H oder $C_1$-$C_4$-Alkyl bedeutet;
$R^2$ H oder $C_1$-$C_4$-Alkyl bedeutet
Y eine direkte Bindung, O oder S darstellt;
m eine ganze Zahl von 1 bis 4 bedeutet;
n 2 oder 3 ist; und
$R^3$ 1- oder 2-Naphthyl bedeutet oder eine Gruppe der Formel:

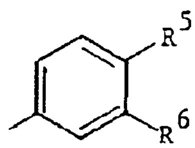

,

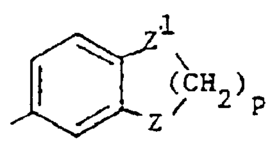

oder Het

worin $R^5$ und $R^6$ jeweils unabhängig voneinander H, $C_1$-$C_4$-Alkyl, $C_1$-$C_4$-Alkoxy, $-(CH_2)_qOH$, Halogen, Trifluormethyl, Cyano, $-(CH_2)_qNR^7R^8$, $-OCO(C_1$-$C_4$-Alkyl), $-SO_2NH_2$ oder $-CONR^9R^{10}$ bedeuten;
wobei entweder $R^7$ und $R^8$ jeweils unabhängig voneinander H oder $C_1$-$C_4$-Alkyl darstellen, oder $R^7$ H bedeutet und $R^8$ $-SO_2(C_1$-$C_4$-Alkyl), $-CONR^9R^{10}$, $-CO(C_1$-$C_4$-Alkyl) oder $-SO_2NH_2$ darstellt;
$R^9$ und $R^{10}$ jeweils unabhängig voneinander H oder $C_1$-$C_4$-Alkyl bedeuten;
q 0, 1 oder 2 darstellt;
Z und $Z^1$ jeweils unabhängig voneinander O oder $CH_2$ darstellen;
p 1, 2 oder 3 ist;
und "Het" Pyridyl, Pyrazinyl oder Thienyl bedeutet.

**2.** Verbindung nach Anspruch 1, wobei $R^1$ H darstellt, X N oder CH bedeutet, m 1, 2 oder 3 ist, n 2 ist, $R^2$ Methyl bedeutet und Y eine direkte Bindung oder O darstellt.

**3.** Verbindung nach Anspruch 1 oder 2, worin $R^3$ 2-Naphthyl, Pyridyl oder eine Gruppe der Formel:

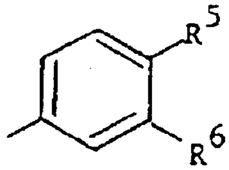

oder

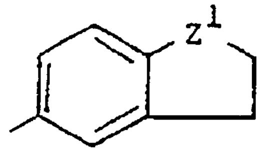

bedeutet, worin $R^5$ und $R^6$ jeweils unabhängig voneinander H, $C_1$-$C_4$-Alkyl, $C_1$-$C_4$-Alkoxy, Halogen, Trifluormethyl, Cyano oder $C_1$-$C_4$-Alkansulfonamido bedeuten und $Z^1$ O oder $CH_2$ darstellt.

4. Arzneimittel, umfassend eine Verbindung der Formel (I) nach einem der vorangehenden Ansprüche oder ein pharmazeutisch verträgliches Salz davon und ein pharmazeutisch verträgliches Verdünnungs- oder Trägermittel.

5. Verbindung der Formel (I) nach einem der Ansprüche 1 bis 3 oder ein pharmazeutisch verträgliches Salz davon, zur Verwendung als Arzneimittel.

6. Verwendung einer Verbindung der Formel (I) nach einem der Ansprüche 1 bis 3 oder eines pharmazeutisch verträglichen Salzes davon zur Herstellung eines Arzneimittels zur Verwendung als Muscarinrezeptorantagonist.

7. Verfahren zur Herstellung einer Verbindung der Formel:

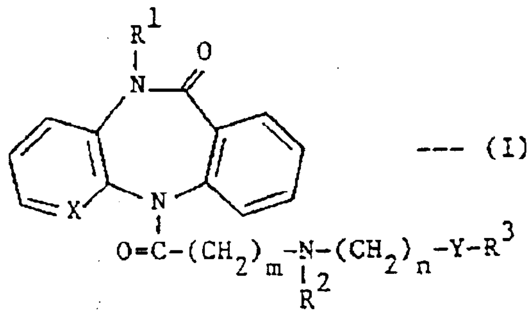

--- (I)

oder eines pharmazeutisch verträglichen Salzes davon,
worin X N oder

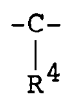

bedeutet, wobei

$R^4$ H, Halogen oder $C_1$-$C_4$-Alkyl darstellt;
$R^1$ H oder $C_1$-$C_4$-Alkyl bedeutet;
$R^2$ H oder $C_1$-$C_4$-Alkyl bedeutet
Y eine direkte Bindung, O oder S darstellt;
m eine ganze Zahl von 1 bis 4 bedeutet;
n 2 oder 3 ist; und
$R^3$ 1- oder 2-Naphthyl bedeutet oder eine Gruppe der Formel:

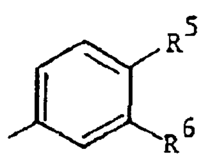

,

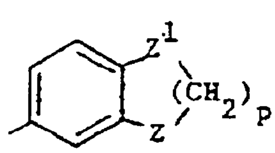

oder Het

worin $R^5$ und $R^6$ jeweils unabhängig voneinander H, $C_1$-$C_4$-Alkyl, $C_1$-$C_4$-Alkoxy, $-(CH_2)_qOH$, Halogen, Trifluormethyl, Cyano, $-(CH_2)_qNR^7R^8$, $-OCO(C_1-C_4\text{-Alkyl})$, $-SO_2NH_2$ oder $-CONR^9R^{10}$ bedeuten;

wobei entweder $R^7$ und $R^8$ jeweils unabhängig voneinander H oder $C_1$-$C_4$-Alkyl bedeuten, oder $R^7$ H bedeutet und $R^8$ $-SO_2(C_1-C_4\text{-Alkyl})$, $-CONR^9R^{10}$, $-CO(C_1-C_4\text{-Alkyl})$ oder $-SO_2NH_2$ darstellt;

$R^9$ und $R^{10}$ jeweils unabhängig voneinander H oder $C_1$-$C_4$-Alkyl bedeuten;

q 0, 1 oder 2 darstellt;

Z und $Z^1$ jeweils unabhängig voneinander O oder $CH_2$ darstellen;

p 1, 2 oder 3 ist;

und "Het" Pyridyl, Pyrazinyl oder Thienyl bedeutet, gekennzeichnet durch entweder:

(a) Umsetzung einer Verbindung der Formel:

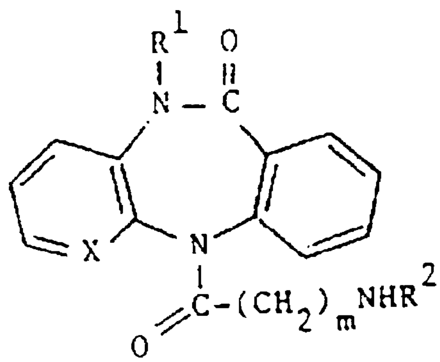

worin $R^1$, $R^2$, X und m wie vorstehend definiert sind, mit einer Verbindung der Formel:

$$Q\text{-}(CH_2)_n\text{-}Y\text{-}R^3 \qquad (III)$$

worin $R^3$, Y und n wie vorstehend definiert sind und Q eine Abgangsgruppe bedeutet,

oder

(b) Umsetzen einer Verbindung der Formel (IV) oder (V)

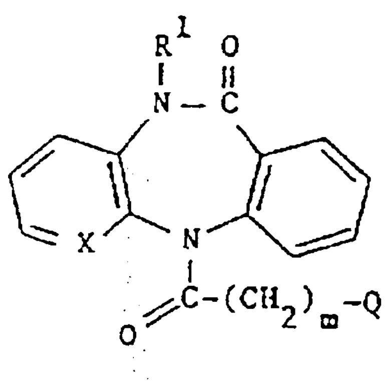

(IV)

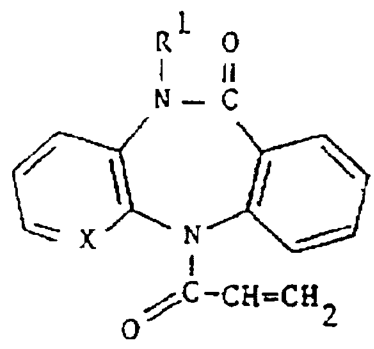

(V)

worin $R^1$, X und m wie vorstehend definiert sind und Q eine Abgangsgruppe bedeutet mit einer Verbindung der Formel:

$R^2NH-(CH_2)_n-Y-R^3$ (VI)

worin $R^2$, $R^3$, Y und n wie vorstehend definiert sind, wobei den Verfahren (a) und (b) gegebenenfalls Umwandeln des Produktes (I) in ein pharmazeutisch verträgliches Salz folgt.

8. Verfahren nach Anspruch 7, **dadurch gekennzeichnet,** daß Q Cl, Br, I oder Methansulfonyloxy bedeutet und daß in (a) und (b) die Umsetzungen in Gegenwart eines Säureakzeptors ausgeführt werden.

9. Verwendung der Verbindungen der Formel (I) zur Herstellung eines Arzneimittels zur Behandlung eines Darmreizungssyndroms, bei einem Patienten, gekennzeichnet durch Verabreichung einer wirksamen Menge einer Verbindung der Formel (I) oder eines pharmazeutisch verträglichen Salzes davon nach einem der Ansprüche 1 bis 3 an den Patienten.

**Patentansprüche für fogende Vertragsstaaten : ES, GR**

1. Verfahren zur Herstellung einer Verbindung der Formel:

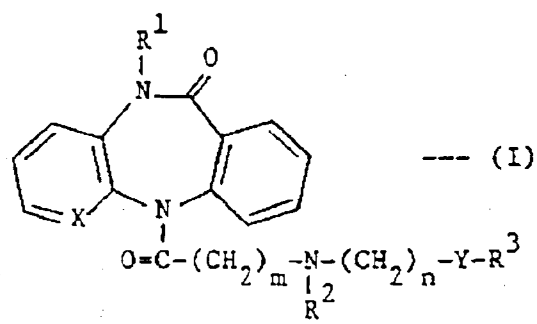

--- (I)

oder eines pharmazeutisch verträglichen Salzes davon,
worin X N oder

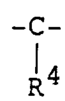

bedeutet, wobei
$R^4$ H, Halogen oder $C_1$-$C_4$-Alkyl darstellt;
$R^1$ H oder $C_1$-$C_4$-Alkyl bedeutet;
$R^2$ H oder $C_1$-$C_4$-Alkyl bedeutet

Y eine direkte Bindung, O oder S darstellt;
m eine ganze Zahl von 1 bis 4 bedeutet;
n 2 oder 3 ist; und
$R^3$ 1- oder 2-Naphthyl bedeutet oder eine Gruppe der Formel:

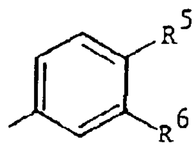

,

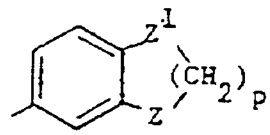

oder Het

worin $R^5$ und $R^6$ jeweils unabhängig voneinander H, $C_1$-$C_4$-Alkyl, $C_1$-$C_4$-Alkoxy, $-(CH_2)_qOH$, Halogen, Trifluormethyl, Cyano, $-(CH_2)_qNR^7R^8$, $-OCO(C_1$-$C_4$-Alkyl), $-SO_2NH_2$ oder $-CONR^9R^{10}$ bedeuten;
wobei entweder $R^7$ und $R^8$ jeweils unabhängig voneinander H oder $C_1$-$C_4$-Alkyl darstellen, oder
$R^7$ H bedeutet und $R^8$ $-SO_2(C_1$-$C_4$- Alkyl), $-CONR^9R^{10}$, $-CO(C_1$-$C_4$-Alkyl) oder $-SO_2NH_2$ darstellt;
$R^9$ und $R^{10}$ jeweils unabhängig voneinander H oder $C_1$-$C_4$-Alkyl bedeuten;
q 0, 1 oder 2 darstellt;
Z und $Z^1$ jeweils unabhängig voneinander O oder $CH_2$ darstellen;
p 1, 2 oder 3 ist;
und "Het" Pyridyl, Pyrazinyl oder Thienyl bedeutet, gekennzeichnet durch entweder:

(a) Umsetzung einer Verbindung der Formel:

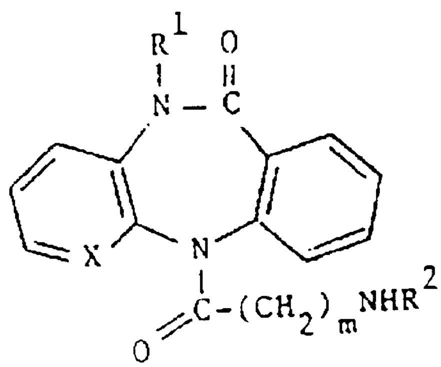

worin $R^1$, $R^2$, X und m wie vorstehend definiert sind, mit einer Verbindung der Formel:

$$Q-(CH_2)_n-Y-R^3 \qquad (III)$$

worin $R^3$, Y und n wie vorstehend definiert sind und Q eine Abgangsgruppe bedeutet,
oder

(b) Umsetzen einer Verbindung der Formel (IV) oder (V)

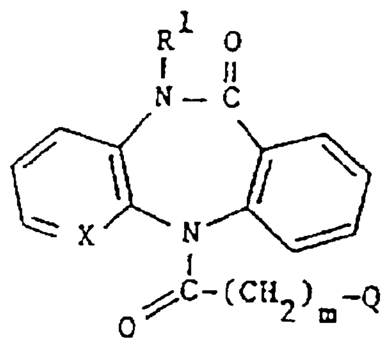

(IV)

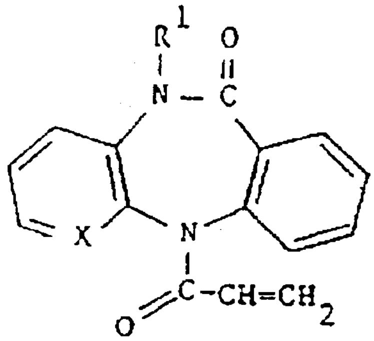

(V)

worin $R^1$, X und m wie vorstehend definiert sind und Q eine Abgangsgruppe bedeutet mit einer Verbindung der Formel:

$$R^2NH\text{-}(CH_2)_n\text{-}Y\text{-}R^3 \qquad (VI)$$

worin $R^2$, $R^3$, Y und n wie vorstehend definiert sind, wobei den Verfahren (a) und (b) gegebenenfalls Umwandeln des Produktes (I) in ein pharmazeutisch verträgliches Salz folgt.

2. Verfahren nach Anspruch 1, **dadurch gekennzeichnet,** daß Q Cl, Br, I oder Methansulfonyloxy bedeutet und daß in (a) und (b) die Umsetzungen in Gegenwart eines Säureakzeptors ausgeführt werden.

3. Verfahren nach Anspruch 1 oder 2, **dadurch gekennzeichnet,** daß es verwendet wird zur Herstellung einer Verbindung der Formel (I), worin $R^1$ H bedeutet, X N oder CH darstellt, m 1, 2 oder 3 bedeutet, n 2 ist, $R^2$ Methyl bedeutet, Y eine direkte Bindung oder O darstellt und $R^3$ 2-Naphthyl, Pyridyl oder eine Gruppe der Formel:

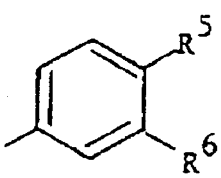

oder

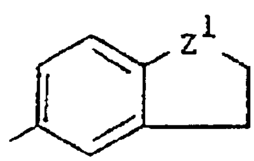

darstellt, worin $R^5$ und $R^6$ jeweils unabhängig voneinander H, $C_1$-$C_4$-Alkyl, $C_1$-$C_4$-Alkoxy, Halogen, Trifluormethyl, Cyano oder $C_1$-$C_4$-Alkansulfonamido bedeuten und $Z^1$ O oder $CH_2$ darstellt.

## Revendications

### Revendications pour les Etats contractants suivants : AT, BE, CH, DE, DK, FR, GB, IT, LI, LU, NL, SE

1. Composé de formule :

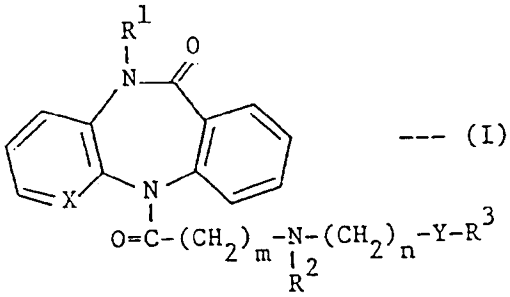

--- (I)

ou sel pharmaceutiquement acceptable d'un tel composé,

dans lequel : X représente N ou $-C(R^4)-$ où

$R^4$ représente H, halogéno ou alkyle en $C_1$-$C_4$ ;

$R^1$ représente H ou alkyle en $C_1$-$C_4$ ;

$R^2$ représente H ou alkyle en $C_1$-$C_4$ ;

Y représente une liaison directe, O ou S ;

m est un nombre entier compris entre 1 et 4 ;

n représente 2 ou 3 ;

et $R^3$ est un groupe 1- ou 2-naphtyle ou un groupe de formule :

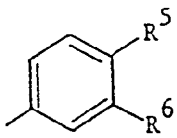

,

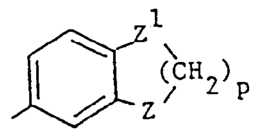

ou Het

où $R^5$ et $R^6$ représentent, chacun indépendamment, H, alkyle en $C_1$-$C_4$, alcoxy en $C_1$-$C_4$, $-(CH_2)_qOH$, halogéno, trifluorométhyle, cyano, $-(CH_2)_qNR^7R^8$, -OCO(alkyle en $C_1$-$C_4$), $-SO_2NH_2$ ou $-CONR^9R^{10}$ ;

où soit $R^7$ et $R^8$ représentent chacun indépendamment H ou alkyle en $C_1$-$C_4$, soit $R^7$ représente H et $R^8$ représente $-SO_2$(alkyle en $C_1$-$C_4$), $-CONR^9R^{10}$, -CO(alkyle en $C_1$-$C_4$) ou $-SO_2NH_2$ ;

$R^9$et $R^{10}$ représentent chacun indépendamment H ou alkyle en $C_1$-$C_4$ ;

q représente 0, 1 ou 2 .

Z et $Z^1$ représentent chacun indépendamment O ou $CH_2$ ;

p représente 1, 2 ou 3 ;

et "Het" est un groupe pyridyle, pyrazinyle ou thiényle.

2. Composé selon la revendication 1, dans lequel $R^1$ représente H, X représente N ou CH, m représente 1, 2 ou 3, n représente 2, $R^2$ représente méthyle et Y représente une liaison directe ou O.

3. Composé selon la revendication 1 ou 2, dans lequel $R^3$ est un groupe 2-naphtyle, pyridyle ou un groupe de formule :

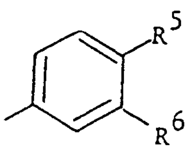

ou

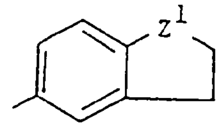

où $R^5$ et $R^6$ représentent chacun indépendamment H, alkyle en $C_1$-$C_4$, alcoxy en $C_1$-$C_4$, halogéno, trifluorométhyle, cyano ou (alcane en $C_1$-$C_4$)sulfonamido, et $Z^1$ représenté O ou $CH_2$.

4. Composition pharmaceutique comprenant un composé de formule (I) selon l'une quelconque des revendications précédentes, ou un sel pharmaceutiquement acceptable d'un tel composé, et un diluant ou un véhicule pharmaceutiquement acceptable.

5. Composé de formule (I) selon l'une quelconque des revendications 1 à 3, ou un sel pharmaceutiquement acceptable d'un tel composé, pour une utilisation comme médicament.

6. Utilisation d'un composé de formule (I) selon l'une quelconque des revendications 1 à 3, ou d'un sel pharmaceutiquement acceptable d'un tel composé, pour la fabrication d'un médicament destiné à être utilisé comme antagoniste de récepteur muscarinique.

7. Procédé de préparation d'un composé de formule :

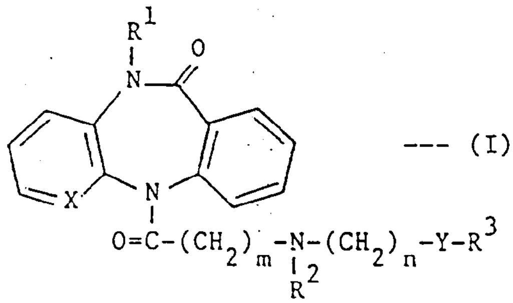

--- (I)

ou d'un sel pharmaceutiquement acceptable d'un tel composé, formule

dans laquelle : X représente N ou $-C(R^4)-$ où

$R^4$ représente H, halogéno ou alkyle en $C_1$-$C_4$ ;

$R^1$ représente H ou alkyle en $C_1$-$C_4$ ;

$R^2$ représente H ou alkyle en $C_1$-$C_4$ ;

Y représente une liaison directe, O ou S ;

m est un nombre entier compris entre 1 et 4 ;

n représente 2 ou 3 ;

et $R^3$ est un groupe 1- ou 2-naphtyle ou un groupe de formule :

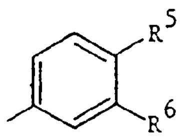

,

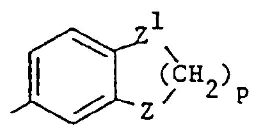

ou Het

où $R^5$ et $R^6$ représentent chacun indépendamment H, alkyle en $C_1$-$C_4$, alcoxy en $C_1$-$C_4$, $-(CH_2)_qOH$, halogéno, trifluorométhyle, cyano, $-(CH_2)_qNR^7R^8$, -OCO(alkyle en $C_1$-$C_4$), $-SO_2NH_2$ ou $-CONR^9R^{10}$ ;

où soit $R^7$ et $R^8$ représentent chacun indépendamment H ou alkyle en $C_1$-$C_4$, soit $R^7$ représente H et $R^8$ représente $-SO_2$(alkyle en $C_1$-$C_4$), $-CONR^9R^{10}$, -CO(alkyle en $C_1$-$C_4$) ou $SO_2NH_2$ ;

$R^9$ et $R^{10}$ représentent chacun indépendamment H ou alkyle en $C_1$-$C_4$ ;

q représente 0, 1 ou 2 ;

Z et $Z^1$ représentent chacun indépendamment O ou $CH_2$ ;

p représente 1, 2 ou 3 ;

et "Het" représente un groupe pyridyle, pyrazinyle ou thiényle ;

caractérisé en ce qu'il consiste :

(a) soit à faire réagir un composé de formule :

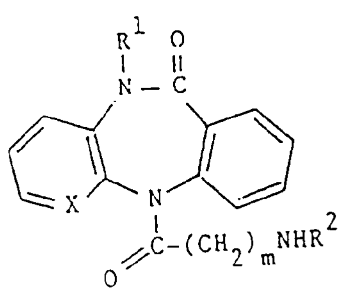

--- (II)

où $R^1$, $R^2$; X et m sont tels que définis ci-dessus, avec un composé de formule :

$$Q-(CH_2)_n-Y-R^3 \quad (III)$$

où $R^3$, Y et n sont tels que définis ci-dessus et Q est un groupe labile,

(b) soit à faire réagir un composé de formule (IV) ou (V)

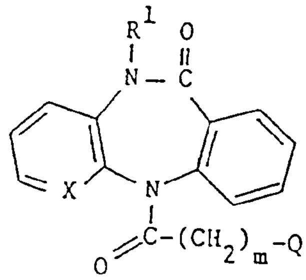

(IV)

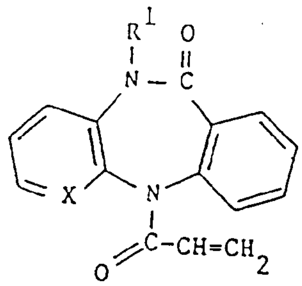

(V)

où $R^1$ , X et m sont tels que définis ci-dessus et Q est un groupe labile, avec un composé de formule :

$$R^2NH-(CH_2)_n-Y-R^3 \quad (VI)$$

où $R^2$, $R^3$, Y et n sont tels que définis ci-dessus, lesdits procédés (a) et (b) étant suivis facultativement par la transformation du composé (I) en un sel pharmaceutiquement acceptable.

8. Procédé selon la revendication 7, caractérisé en ce que Q représente Cl, Br, I ou méthanesulfonyloxy, et en ce que, sous (a) et (b), les réactions sont mises en oeuvre en présence d'un accepteur d'acide.

9. Utilisation des composés de formule (I) pour la préparation d'un médicament pour le traitement du syndrome d'intestin irritable chez un patient nécessitant un tel traitement, caractérisée par l'administration audit patient d'une quantité efficace d'un composé de formule (I) ou d'un sel pharmaceutiquement acceptable d'un tel composé selon l'une quelconque des revendications 1 à 3.

**Revendications pour les Etats contractants suivants : ES, GR**

1. Procédé de préparation d'un composé de formule :

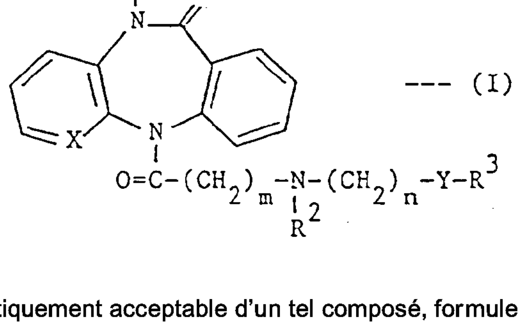

--- (I)

ou d'un sel pharmaceutiquement acceptable d'un tel composé, formule dans laquelle : X représente N ou -C($R^4$)- où

$R^4$ représente H, halogéno ou alkyle en $C_1$-$C_4$ ;
$R^1$ représente H ou alkyle en $C_1$-$C_4$ ;
$R^2$ représente H ou alkyle en $C_1$-$C_4$ ;
Y représente une liaison directe, O ou S ;
m est un nombre entier compris entre 1 et 4 ;
n représente 2 ou 3 ;

et $R^3$ est un groupe 1- ou 2-naphtyle ou un groupe de formule :

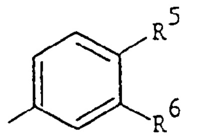
,
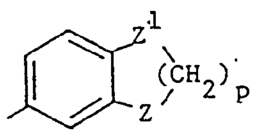

ou Het

où $R^5$ et $R^6$ représentent chacun indépendamment H, alkyle en $C_1$-$C_4$, alcoxy en $C_1$-$C_4$, $-(CH_2)_qOH$, halogéno, trifluorométhyle, cyano, $-(CH_2)_qNR^7R^8$, -OCO(alkyle en $C_1C_4$), $-SO_2NH_2$ ou $-CONR^9R^{10}$ ;

où soit $R^7$ et $R^8$ représentent chacun indépendamment H ou alkyle en $C_1$-$C_4$, soit $R^7$ représente H et $R^8$ représente $-SO_2$(alkyle en $C_1$-$C_4$), $-CONR^9R^{10}$, -CO(alkyle en $C_1$-$C_4$) ou $-SO_2NH_2$ ;
$R^9$ et $R^{10}$ représentent chacun indépendamment H ou alkyle en $C_1$-$C_4$ ;
q représente 0, 1 ou 2 ;
Z et $Z^1$ représentent chacun indépendamment O ou $CH_2$ ;
p représente 1, 2 ou 3 ;

et "Het" représente un groupe pyridyle, pyrazinyle ou thiényle ;

caractérisé en ce qu'il consiste :

(a) soit à faire réagir un composé de formule :

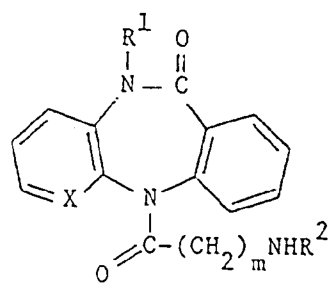

--- (II)

où $R^1$, $R^2$, X et m sont tels que définis ci-dessus, avec un composé de formule :

$$Q\text{-}(CH_2)_n\text{-}Y\text{-}R^3 \qquad (III)$$

où $R^3$ , Y et n sont tels que définis ci-dessus et Q est un groupe labile,

(b) soit à faire réagir un composé de formule (IV) ou (V)

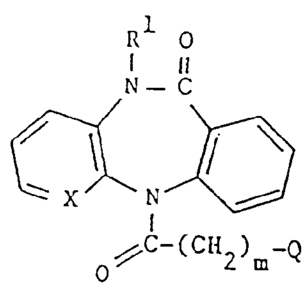

(IV)

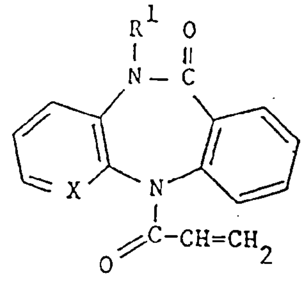

(V)

où $R^1$, X et m sont tels que définis ci-dessus et Q est un groupe labile, avec un composé de formule :

$$R^2NH-(CH_2)_n-Y-R^3 \qquad (VI)$$

où $R^2$, $R^3$, Y et n sont tels que définis ci-dessus, lesdits procédés (a) et (b) étant suivis facultativement par la transformation du composé (I) en un sel pharmaceutiquement acceptable.

2. Procédé selon la revendication 1, caractérisé en ce que Q représente Cl, Br, I ou méthanesulfonyloxy, et en ce que, sous (a) et (b), les réactions sont mises en oeuvre en présence d'un accepteur d'acide.

3. Procédé selon la revendication 1 ou 2, caractérisé en ce qu'il est utilisé pour préparer un composé de formule (I) dans lequel $R^1$ représente H, X représente N ou CH, m représente 1, 2 ou 3, n représente 2, $R^2$ représente méthyle, Y représente une liaison directe ou O, et $R^3$ est un groupe 2-naphtyle, pyridyle ou un groupe de formule :

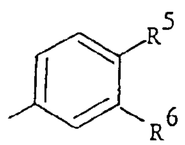

ou

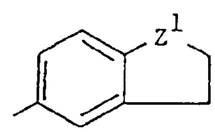

où $R^5$ et $R^6$ représentent chacun indépendamment H, alkyle en $C_1$-$C_4$, alcoxy en $C_1$-$C_4$, halogéno, trifluorométhyle, cyano ou (alcane en $C_1$-$C_4$)sulfonamido, et $Z^1$ représente O ou $CH_2$.